# EUROPÄISCHE PATENTSCHRIFT

(11) **EP 4 382 041 B1**
(45) Veröffentlichungstag und Bekanntmachung des Hinweises auf die Patenterteilung: **05.02.2025**
(21) Anmeldenummer: 22212648.4
(22) Anmeldetag: 09.12.2022
(51) Int. Cl.: A61B 5/0536, A61B 5/00

(54) **MESSGERÄT ZUR BIOIMPEDANZ-SPEKTROSKOPIE DER BRUST**
MEASURING DEVICE FOR BIOIMPEDANCE SPECTROSCOPY OF THE BREAST
APPAREIL DE MESURE POUR LA SPECTROSCOPIE DE BIOIMPÉDANCE DU SEIN

(43) Veröffentlichungstag der Anmeldung: 12.06.2024
(73) Patentinhaber: XERA3 Deutschland GmbH, 22335 Hamburg (DE)
(72) Erfinder: Grabow, Jörg, 07426 Königsee (DE); Schwab, Horst, 01445 Radebeul (DE)
(74) Vertreter: Stolmár & Partner Patentanwälte PartG mbB

(56) Entgegenhaltungen:
- CN-A- 112 568 890
- CN-U- 203 647 341
- US-A1- 2004 210 158
- US-A1- 2010 217 148
- US-A1- 2016 331 266
- MD SHEKH ET AL: "Development of Multi-frequency Electrical Impedance Spectroscopy (EIS) System for Early Detection of Breast Cancer", INTERNATIONAL JOURNAL OF ELECTRONICS & INFORMATICS, vol. 2, no. 1, 18 February 2013 (2013-02-18), XP093043011, ISSN: 2186-0114
- GUTIERREZ-LOPEZ MARCOS ET AL: "Electrical Impedance-Based Methodology for Locating Carcinoma Emulators on Breast Models", JOURNAL OF SENSORS, vol. 2019, 2 May 2019 (2019-05-02), US, pages 1 - 16, XP093042998, ISSN: 1687-725X, Retrieved from the Internet <URL:https://downloads.hindawi.com/journals/js/2019/8587191.pdf> DOI: 10.1155/2019/8587191

## Beschreibung

Die Erfindung betrifft ein Messgerät und ein Verfahren zur Vermessung eines Bioimpedanzprofils von menschlichen Brustgewebe.

Brustkrebs gilt in Deutschland und anderen Ländern als ein Hauptgrund für Invalidität und eine der häufigsten Todesursachen bei Frauen. Rund 72.000 Frauen erkranken in Deutschland jährlich an Brustkrebs, und rund 17.000 Frauen sterben jährlich an dieser Erkrankung.

Die Behandlungsaussichten einer Brustkrebserkrankung sind stark davon abhängig, in welchem Stadium die Erkrankung erkannt wird. Früherkennung zählt daher zu den wirksamsten Methoden, die Krankheit einzudämmen.

Gängige Methoden für die Früherkennung von Brustkrebs sind Tastuntersuchungen, Ultraschalluntersuchungen, und die Mammografie. Während Tastuntersuchungen und Ultraschalluntersuchungen oftmals nicht in der Lage sind, kleine Tumore zuverlässig zu identifizieren, ist die Mammografie für die Patientin äußerst unangenehm, und stellt durch die damit einhergehende Strahlenbelastung und Gewebekompression selbst einen Risikofaktor für eine nachteilige Entwicklung krankhaft veränderten Brustgewebes dar.

Seit einiger Zeit sind Methoden bekannt, die Gewebezusammensetzung eines menschlichen Körpers elektrisch zu vermessen. Dazu werden geringe Wechselströme durch das Körpergewebe geleitet, und die Impedanz des Gewebes bestimmt. Die dabei verwendeten Ströme sind so gering, dass es nicht zu einer Reizung des Gewebes kommt. Dieses als Bioimpedanzmessung bezeichnete Verfahren wird hauptsächlich zur Bestimmung des **Körperfettanteils in der Ernährungsberatung angewendet.** US 2016/331266 A1 sowie MD SHEKH ET AL: "Development of Multi-frequency Electrical Impedance Spectroscopy (EIS) System for Early Detection of Breast Cancer", INTERNATIONAL JOURNAL OF ELECTRONICS & INFORMATICS, Bd. 2, Nr. 1, 18. Februar 2013 (2013-02-18), ISSN: 2186-0114 beschreiben die Vermessung eines Bioimpedanzprofils von menschlichen Gewebe. CN 112568890 A offenbart eine Ansteuerung von vier Elektrode für eine Bioimpedanz-Spektroskopie.

Es besteht daher eine Aufgabe der Erfindung darin, ein alternatives Verfahren bereitzustellen, um Gewebeveränderungen in menschlichen Brustgewebe frühzeitig zu erkennen, sodass rechtzeitig weiterführende differenzialdiagnostische Untersuchungen und gegebenenfalls eine frühzeitige Therapie eingeleitet werden können.

Eine weitere Aufgabe der Erfindung besteht darin, für ein entsprechendes alternatives Verfahren ein geeignetes Messgerät bereitzustellen.

Gemäß eines Aspekts der vorliegenden Erfindung die Aufgabe gelöst durch ein Bioimpedanz-Messgerät zur Vermessung von Brustgewebe gemäß Anspruch 1, mit einem Steuergerät und wenigstens einem Messapplikator, welcher wenigstens vier Kontaktelektroden aufweist, welche über elektrische Leitungen mit dem Steuergerät verbunden oder verbindbar sind, wobei das Steuergerät eingerichtet ist, ein elektrisches Wechselstromsignal zu erzeugen und an zwei der wenigstens vier Kontaktelektroden abzugeben, sodass diese als Stromelektroden fungieren, und ein elektrisches Wechselspannungssignal zwischen zwei der wenigstens vier Kontaktelektroden aufzunehmen, sodass diese als Spannungselektroden fungieren, wobei wenigstens zwei der Kontaktelektroden, die als Spannungselektroden fungieren, nicht gleichzeitig als Stromelektroden fungieren.

Die Erfindung beruht auf der Erkenntnis, dass mittels einer Bioimpedanzmessung des Brustgewebes über geeignet angeordnete Kontaktelektroden eine lokalisierte Vermessung des Brustgewebes möglich ist.

Die Kontaktelektroden des wenigstens einen Messapplikators an den Ecken eines regelmäßigen Polygons angeordnet. Eine entsprechende Anordnung vereinfacht eine reproduzierbare Ausführung der Biompedanzmessung, sodass die Ergebnisse von in zeitlichem Abstand zueinander durchgeführten Messungen einfach verglichen werden können. Dabei weist der Messapplikator vier Kontaktelektroden auf, die an den Ecken eines Quadrats angeordnet sind.

In einer möglichen Ausführungsform umfasst der Messapplikator dabei einen Trägerbereich mit armartigen Fortsätzen, an deren Enden die Kontaktelektroden angeordnet sind. Durch diese Ausführung kann der Messapplikator mit besonders geringer Kontaktfläche auf das zu untersuchende Brustgewebe aufgebracht, beispielsweise aufgelegt werden.

Der Trägerbereich kann dabei eine Öffnung umfassen, die in einem Mittelpunkt des regelmäßigen Polygons angeordnet ist. Der Messapplikator kann so auf das Brustgewebe aufgelegt werden, dass die Mamille durch die Öffnung sichtbar bleibt. Auf diese Weise ist bei wiederholten Messungen eine reproduzierbare Positionierung des Messapplikators leicht zu erreichen.

Der Trägerbereich des Messapplikators kann dabei vorzugsweise aus einem flexiblen Material bestehen, sodass sich der Messapplikator flexibel an das Brustgewebe anlegen kann. Ein geeignetes Material mit hoher Biokompatibilität ist beispielsweise Silikon.

In einer bevorzugten Ausführungsform sind dabei die Kontaktelektroden lösbar mit dem Messapplikator verbunden. So können beispielsweise einmalig verwendbare Kontaktelektroden mit einem wiederverwendbaren Messapplikator verwendet werden.

Das Steuergerät des Bioimpedanz-Messgeräts ist in einer möglichen Ausgestaltung eingerichtet, das Wechselstromsignal mit einer veränderlichen Frequenz zu erzeugen. Durch die Vermessung der Bioimpedanz bei unterschiedlichen Frequenzen ist eine genauere Charakterisierung des zu vermessenden Brustgewebes möglich.

Das Steuergerät ist weiterhin vorzugsweise eingerichtet, wechselweise unterschiedliche Kontaktelektroden als Spannungselektroden anzusteuern. Durch die wechselweise unterschiedliche Ansteuerung der Kontaktelektroden lässt sich ein ortsaufgelöstes Bioimpedanzprofil des zu untersuchenden Gewebes erstellen.

Ein Bioimpedanz-Messgerät kann in einer vorteilhaften Ausgestaltung der Erfindung weiterhin eine Recheneinheit umfassen, die eingerichtet ist, aus den bei verschiedenen Frequenzen und/oder mit verschiedenen Elektrodenkonfigurationen aufgenommenen Wechselspannungssignalen eine räumliche Impedanzverteilung des untersuchten Brustgewebes zu berechnen.

Das Steuergerät umfasst dabei vorzugsweise eine Speichereinrichtung, und ist eingerichtet, die berechnete Impedanzverteilung und/oder die bei verschiedenen Frequenzen und/oder mit verschiedenen Elektrodenkonfigurationen aufgenommenen Wechselspannungssignale zu speichern.

In einer möglichen Ausführung ist das Steuergerät des Bioimpedanz-Messgerät in einem fahrbaren Gehäuse angeordnet. Es kann somit leicht zu einer Untersuchungsliege, auf welcher die zu untersuchende Person liegt, positioniert werden. Als fahrbares Gehäuse kann beispielsweise ein medizinischer Gerätewagen Verwendung finden.

Das fahrbare Gehäuse kann dabei einen Auslegearm umfassen, entlang dessen die elektrischen Leitungen geführt oder führbar sind. Hierdurch kann vermieden werden, dass die elektrischen Leitungen auf der Hautoberfläche der zu untersuchenden Person liegen.

In einer besonders vorteilhaften Ausführung kann das Bioimpedanz-Messgerät zwei Messapplikatoren umfassen, mit welchen beide Brüste einer zu untersuchenden Person gleichzeitig vermessen werden können.

Die Aufgabe wird weiterhin gelöst durch ein Verfahren zur Aufnahme eines Bioimpedanzprofils von menschlichen Brustgewebe gemäß Anspruch 15, mit den Schritten: applizieren eines Messapplikators mit vier Kontaktelektroden auf das zu untersuchende Brustgewebe, einspeisen eines Wechselstromsignals über zwei der vier Kontaktelektroden, die als Stromelektroden fungieren, und aufnehmen eines Wechselspannungssignals über zwei der vier Kontaktelektroden, die als Spannungselektroden fungieren, wobei eine Frequenz des Wechselstromsignals über einen vorgegebenen Frequenzbereich variiert wird, und wobei eine Funktion jeder der vier Kontaktelektroden zwischen Stromelektrode und Spannungselektroden gewechselt wird. Durch das erfindungsgemäße Verfahren ist es möglich, ein ortsaufgelöstes Bioimpedanzprofil des Brustgewebes zu erstellen.

Dabei umfasst der Messapplikator vier an den Ecken eines Quadrats angeordnete Kontaktelektroden, wobei in einem ersten Messintervall eine erste und eine zweite Kontaktelektrode als Stromelektrode fungieren, und eine dritte und eine vierte Kontaktelektrode als Spannungselektroden fungieren, in einem zweiten Messintervall die erste und die dritte Kontaktelektrode als Stromelektrode fungieren, und die zweite und die vierte Kontaktelektrode als Spannungselektroden fungieren, in einem dritten Messintervall die erste und die vierte Kontaktelektrode als Stromelektrode fungieren, und die zweite und die dritte Kontaktelektrode als Spannungselektroden fungieren, in einem vierten Messintervall die zweite und die dritte Kontaktelektrode als Stromelektronen fungieren, und die erste und die vierte Kontaktelektrode als Spannungselektroden fungieren, in einem fünften Messintervall die zweite und die vierte Kontaktelektrode als Stromelektrode fungieren, und die erste und die dritte Kontaktelektrode als Spannungselektroden fungieren, und in einem sechsten Messintervall die dritte und die vierte Kontaktelektrode als Stromelektrode fungieren, und die erste und die zweite Kontaktelektrode als Spannungselektroden fungieren.

In einer weiteren bevorzugten Ausführung des erfindungsgemäßen Verfahrens wird die Frequenz des Wechselstromsignals in jedem Messintervall in mehreren Frequenzschritten in einem Frequenzbereich zwischen 1 kHz und 1 MHz variiert.

In jedem der Messintervalle und bei jedem Frequenzschritt können dabei eine Amplitude und eine Phase des Spannungssignals gemessen werden. Auf diese Weise kann ein orts-und frequenzaufgelöstes Bioimpedanzprofil des zu untersuchenden Gewebes erstellt werden. Dazu werden die aufgenommenen Messwerte abgespeichert.

Ein so gespeichertes Bioimpedanzprofil kann mit einem zuvor aufgenommenen Bioimpedanzprofil des selben Gewebes verglichen werden. Das zuvor aufgenommene Bioimpedanzprofil kann beispielsweise bei einer vorangehenden regelmäßigen Vorsorgeuntersuchung aufgenommen worden sein.

Wenn der Vergleich des Bioimpedanzprofils mit dem zuvor aufgenommenen Bioimpedanzprofil eine Änderung ergibt, welche ein vorgegebenes Maß übersteigt, so kann ein Veränderungssignal erzeugt werden. Dieses Veränderungssignal kann die Notwendigkeit anzeigen, weitere differenzialdiagnostische Schritte einzuleiten, um eine gegebenenfalls vorliegende krankhafte Gewebeveränderung auszuschließen oder zu bestätigen.

Die Erfindung wird nachfolgend anhand einiger beispielhafter Darstellungen näher erläutert. Dabei dienen die Darstellungen ausschließlich zum besseren Verständnis der Erfindung, ohne diese einzuschränken.

Es zeigen:
Figur 1: eine Prinzipdarstellung der Bioimpedanzmessung von Brustgewebe,
Figur 2A-2F: Verbindungskonfigurationen der Kontakelektroden,
Figur 3A-3F: Untersuchungsbereiche der Verbindungskonfigurationen aus den Figuren 2A-2F,
Figur 4: einen Messapplikator,
Figur 5: ein Bioimpedanz-Messsystem,
Figur 6: ein Untersuchungssystem.

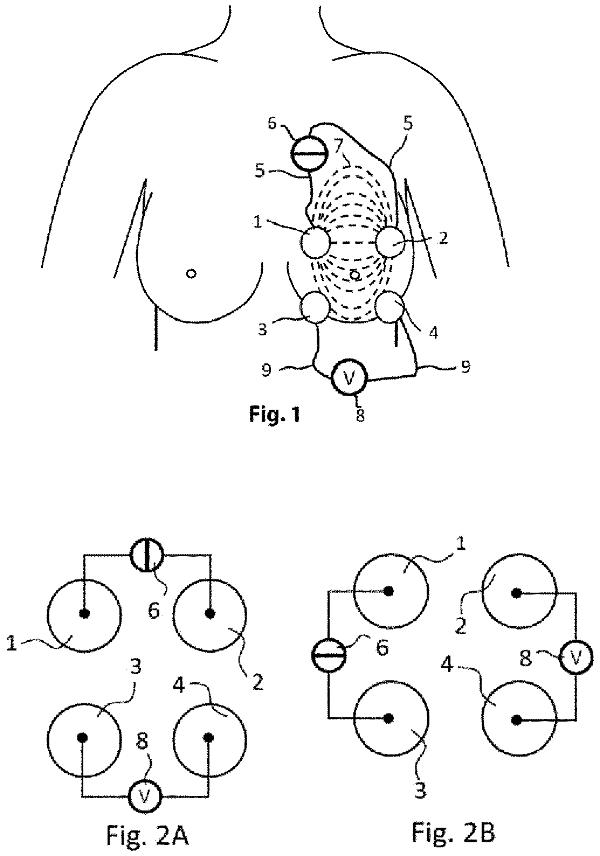

Figur 1 zeigt einen weiblichen Torso mit angedeutetem Brustgewebe, welches einer Bioimpedanzmessung unterzogen werden soll. Dazu sind auf die linke Brust vier Kontaktelektroden 1, 2, 3, 4 aufgebracht. Die Kontaktelektroden 1, 2 sind über elektrische Leitungen 5 mit einer Wechselstromquelle 6 verbunden, sie fungieren daher als Stromelektroden. Über die Wechselstromquelle 6 wird ein Stromsignal in Form eines schwachen hochfrequenten Wechselstroms in das zu untersuchende Brustgewebe eingeleitet. Dadurch entsteht in dem Brustgewebe ein elektromagnetisches Feld, welches durch die Feldlinien 7 angedeutet ist. Durch das elektromagnetische Feld entsteht zwischen den Kontaktelektroden 3, 4 eine Potenzialdifferenz, welche durch das Spannungsmessgerät 8 gemessen wird. Das Spannungsmessgerät 8 ist über elektrische Leitungen 9 mit den Kontaktelektroden 3, 4 verbunden, diese fungieren daher als Spannungselektroden.

Der Verlauf der elektromagnetischen Feldlinien 7, welcher in Figur 1 stark vereinfacht dargestellt ist, hängt von der Frequenz des eingeprägten Wechselstroms und von der Zusammensetzung des Brustgewebes im Bereich zwischen den Stromelektroden 1, 2 und den Spannungselektroden 3, 4 ab. Durch Veränderung der Frequenz des eingeprägten Wechselstroms kann die Zusammensetzung des Brustgewebes im Messbereich bestimmt werden.

Für die hier beschriebene Bioimpedanzmessung ist es nicht erforderlich, die physiologische Gewebezusammensetzung tatsächlich zu bestimmen. Vielmehr ist es ausreichend, den Verlauf der Amplitude und der Phase des Spannungssignals in Abhängigkeit von der Frequenz des Stromsignals aufzuzeichnen. Dies liegt daran, dass es für eine frühzeitige Erkennung von Gewebeveränderungen ausreichend ist, eine Veränderung der physiologischen Gewebezusammensetzung zu erkennen, welche sich eben in einem veränderten Verlauf der Amplitude und der Phase des Spannungssignals zeigt.

Die Figuren 2A bis 2F zeigen, wie bei der Aufnahme des Impedanzprofils die Kontaktelektroden 1, 2, 3, 4 nacheinander mit der Wechselstromquelle 6 und mit dem Spannungsmessgerät 8 verbunden werden.

Figur 2A zeigt dabei die schon in Figur 1 angedeutete Konfiguration, in welcher die Kontaktelektroden 1 und 2 als Stromelektroden mit der Wechselstromquelle 6 verbunden sind, während die Kontaktelektroden 3 und 4 als Spannungselektroden mit dem Spannungsmessgerät 8 verbunden sind.

Figur 2B zeigt eine Konfiguration, in welcher die Kontaktelektroden 1 und 3 als Stromelektroden mit der Wechselstromquelle 6 verbunden sind, während die Kontaktelektroden 2 und 4 als Spannungselektroden mit dem Spannungsmessgerät 8 verbunden sind.

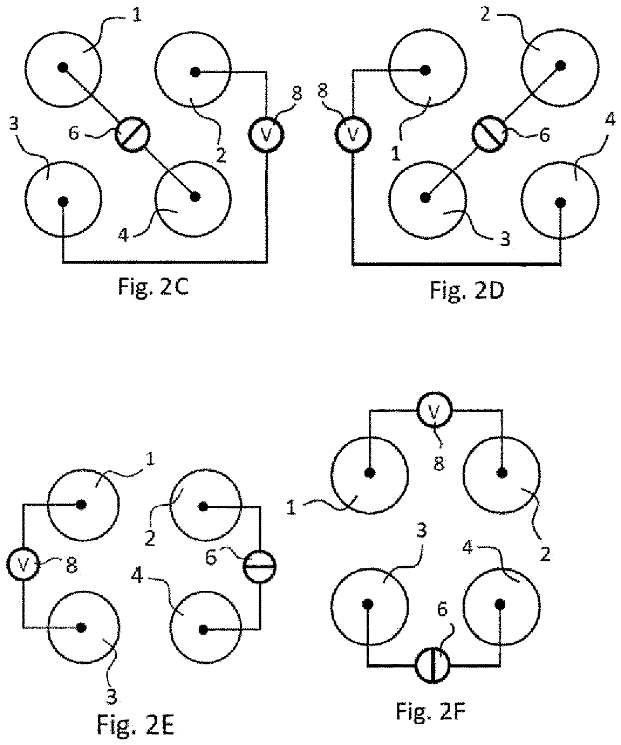

Figur 2C zeigt eine Konfiguration, in welcher die Kontaktelektroden 1 und 4 als Stromelektroden mit der Wechselstromquelle 6 verbunden sind, während die Kontaktelektroden 2 und 3 als Spannungselektroden mit dem Spannungsmessgerät 8 verbunden sind.

Figur 2D zeigt eine Konfiguration, in welcher die Kontaktelektroden 2 und 3 als Stromelektroden mit der Wechselstromquelle 6 verbunden sind, während die Kontaktelektroden 1 und 4 als Spannungselektroden mit dem Spannungsmessgerät 8 verbunden sind.

Figur 2E zeigt eine Konfiguration, in welcher die Kontaktelektroden 2 und 4 als Stromelektronen mit der Wechselstromquelle 6 verbunden sind, während die Kontaktelektroden 1 und 3 als Spannungselektroden mit dem Spannungsmessgerät 8 verbunden sind.

Figur 2F zeigt eine Konfiguration, in welche die Kontaktelektroden 3 und 4 als Stromelektrode mit der Wechselstromquelle 6 verbunden sind, während die Kontaktelektroden 1 und 2 als Spannungselektroden mit dem Spannungsmessgerät 8 verbunden sind.

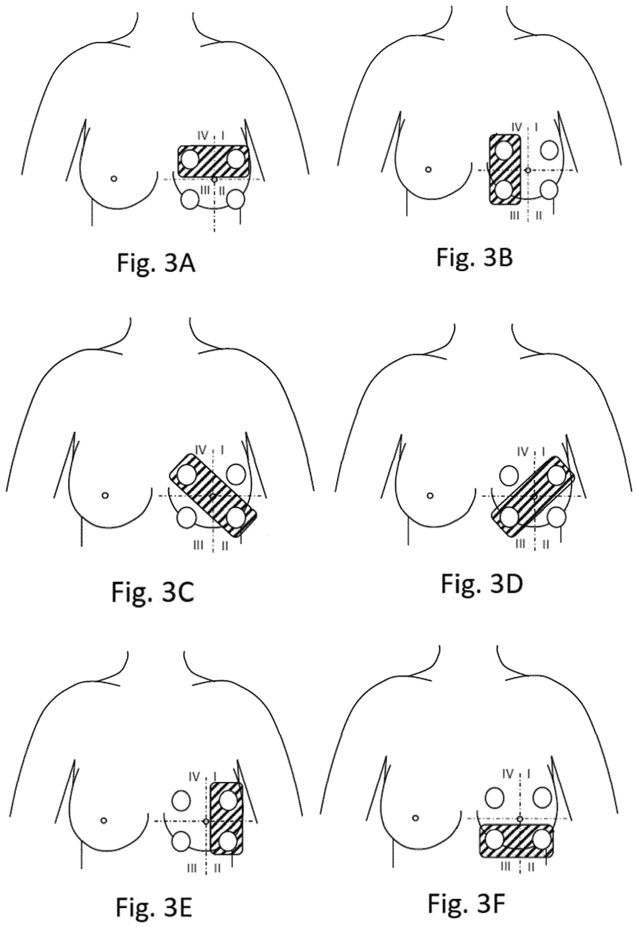

In den Figuren 3A bis 3F sind die Bereiche des Brustgewebes dargestellt, welche jeweils mit den in den Figuren 2A bis 2F dargestellten Konfigurationen der Kontaktelektroden von dem Wechselstromsignal durchflossen werden. Dazu sind die vier Sektoren der Brust mit I-IV gekennzeichnet.

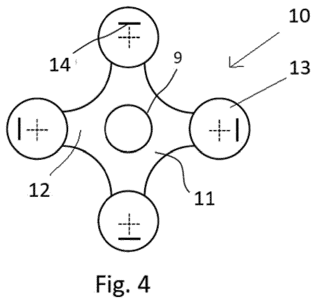

In Figur 4 ist ein Messapplikator 10 eines erfindungsgemäßen Bioimpedanz-Messgeräts dargestellt. Der Messapplikator umfasst einen Trägerbereich 11, welche vier armartige Fortsätze 12 aufweist, an deren Enden Aufnahmen 13 für Kontaktelektroden angeordnet sind. Über Schlitze 14 in den Aufnahmen 13 können Kontaktelektroden, die in Figur 4 nicht dargestellt sind, in die Aufnahmen 13 eingeführt werden, so dass sie beim Auflegen des Messapplikators 10 auf das zu untersuchende Brustgewebe mit diesem in Kontakt kommen. Der Trägerbereich 11 mit den Fortsätzen 12 ist aus einem flexiblen und biokompatiblen Material wie Silikon gefertigt. Die Fortsätze 12 legen sich daher beim Auflegen des Messapplikators 10 auf das zu untersuchende Brustgewebe schonend an dieses an, ohne dass es notwendig ist, Druck auf den Messapplikator 10 auszuüben.

In der Mitte des Messapplikators 10 ist eine Öffnung 15 vorgesehen, durch welche beim Auflegen des Messapplikators 10 auf das Brustgewebe die Mamille sichtbar bleibt. Es wird dadurch vereinfacht, bei aufeinanderfolgenden Messungen den Messapplikator 10 immer in gleicher Position auf das zu untersuchende Brustgewebe aufzulegen.

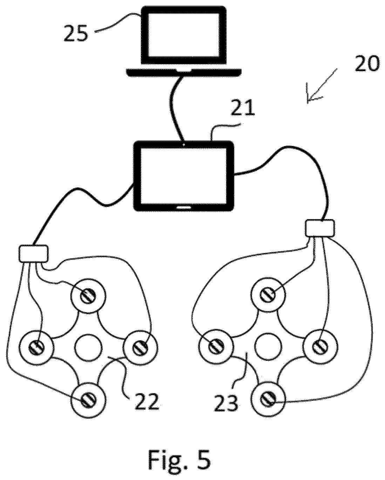

In Figur 5 ist ein Bioimpedanz-Messsystem 20 dargestellt. Das Bioimpedanz Messsystem 20 umfasst ein Steuergerät 21, welches eine Wechselstromquelle und ein Spannungsmessgerät enthält, die der Übersichtlichkeit halber nicht separat dargestellt sind. An das Steuergerät 21 sind zwei Messapplikatoren 22, 23 angeschlossen, welche wie der in Figur 4 dargestellte Messapplikator 10 ausgeführt sind. Mit den zwei Messapplikatoren 22, 23 können beide Brüste einer zu untersuchenden Person gleichzeitig vermessen werden.

Das Bioimpedanz-Messsystem 20 ist zusätzlich mit einem Computer 25 verbunden, mittels dem die bei verschiedenen Frequenzen des Wechselstromsignals und bei verschiedenen Elektrodenkonfigurationen aufgenommenen Phasen und Amplituden des Spannungssignals protokolliert und zu einer räumlichen Impedanzverteilung des untersuchten Brustgewebes ausgewertet werden. Unter einer räumlichen Impedanzverteilung ist hier nicht zwangsläufig eine genaue ortsaufgelöste Impedanzverteilung zu verstehen bei welcher im Sinne einer tomographischen Schichtaufnahme für jedes Raumelement des untersuchten Brustgewebes ein separater Impedanz Messwert vorliegt. Unter einer räumlichen Impedanzverteilung im Sinne der vorliegenden Erfindung ist auch ein mehrdimensionales Kennlinienfeld zu verstehen, in welchem über die unterschiedlichen Elektrodenkonfigurationen und unterschiedlichen Messfrequenzen jeweils Amplitude und Phase des Spannungssignals aufgetragen sind.

Das Steuergerät 21 kann beispielsweise für jede Elektrodenkonfiguration die Frequenz des Wechselstromsignals in mehreren Schritten zwischen 1 kHz und 1 MHz variieren. Die Frequenz kann beispielsweise jeweils in Schritten von 1 kHz erhöht werden, sodass nach 1000 Schritten die Frequenz von 1 MHz erreicht ist. Hierbei reicht für jeden Frequenzschritt eine kurze Messzeit von beispielsweise 0,1 Sekunden aus, um Messwerte für die Phase und die Amplitude der Wechselspannung zu bestimmen. Für die 1000 Frequenzschritte werden somit weniger als 2 Minuten benötigt, sodass die komplette Vermessung des Brustgewebes mit allen 6 Elektrodenkonfigurationen in etwa 10 Minuten abgeschlossen werden kann.

Eine Auswertung der Messergebnisse kann unmittelbar nach der Messung durch den Computer 25 erfolgen. Zur Auswertung der Messergebnisse können zum einen Referenzwerte herangezogen werden, welche normale Verläufe der Messwerte bei gesundem Gewebe repräsentieren. Da solche Referenzwerte allerdings nur grobe Richtwerte darstellen können, ist es sinnvoll, die Messergebnisse eine Untersuchung jeweils mit Messergebnissen einer vorangegangenen Untersuchung des selben Brustgewebes zu vergleichen. Wenn beispielsweise eine erfindungsgemäße Bioimpedanzmessung regelmäßig im Zeitabstand von einem Jahr durchgeführt wird, so lassen sich verdächtige Veränderungen der Gewebezusammensetzung relativ früh erkennen, noch bevor eine tastbare oder per Ultraschalluntersuchung sichtbare Gewebeveränderung vorliegt.

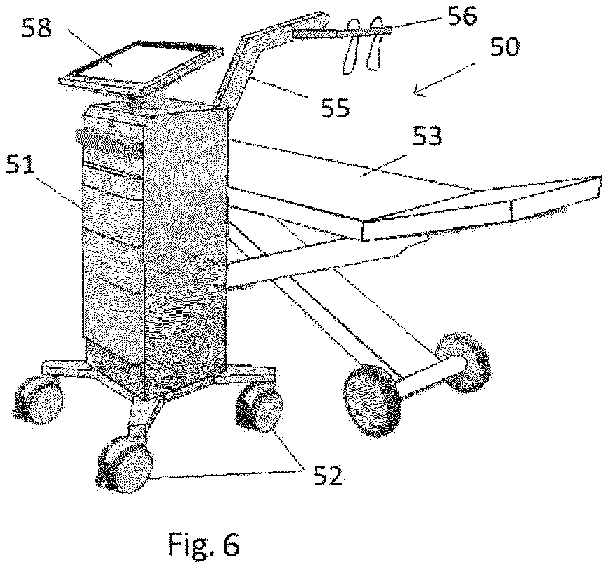

Ein beispielhaftes Untersuchungssystem zur Durchführung der Bioimpedanzmessung gemäß dieser Erfindung ist in Figur 6 dargestellt. Das Untersuchungssystem 50 umfasst einen Gerätewagen 51, in welchem das in Figur 5 dargestellte Bioimpedanz-Messsystem 20 integriert ist. Der Gerätewagen 51 ist mit Rollen 52 versehen, um ihn zu einer Untersuchungsliege 53 zu positionieren, auf welcher die zu untersuchende Person waagerecht positioniert werden kann. An dem Gerätewagen 51 ist weiterhin ein Auslegearm 55 vorgesehen, entlang dem elektrische Leitungen von dem Steuergerät 21, welches in Figur 6 nicht dargestellt ist, verlaufen. Am patientennahen Ende des Auslegearms 55 ist ein Verbindungsbereich 56 vorgesehen, in welchem die elektrischen Leitungen frei zugänglich sind. Dabei sind acht elektrische Leitungen vorgesehen, um zwei Messapplikatoren mit jeweils vier Kontaktelektroden anzuschließen. Der Übersichtlichkeit halber sind in Figur 6 nur zwei Leitungen dargestellt.

Der Auslegearm 55 kann so über der zu untersuchenden Person positioniert werden, dass die in Figur 6 nicht dargestellten Messapplikatoren beidseitig auf das Brustgewebe aufgelegt werden können, ohne dass die elektrischen Leitungen störend auf der Haut der zu untersuchenden Person abgelegt werden müssen. Die freien Enden der elektrischen Leitungen können mit einmal zu verwendenden Kontaktelektroden verbunden werden, welche dann in die jeweiligen Aufnahmen der Messapplikatoren eingeschoben werden.

Ein Bedienpanel 58 an dem Gerätewagen 61 dient zur Bedienung des Bioimpedanz-Messsystems durch einen Benutzer. Das Bedienpanel 58 kann mit dem Computer 25 verbunden sein, oder ein Bestandteil des Computers sein.

## Patentansprüche

1. Bioimpedanz-Messgerät zur Vermessung von Brustgewebe, mit einem Steuergerät und wenigstens einem Messapplikator, welcher eine erste, eine zweite, eine dritte und eine vierte Kontaktelektrode aufweist, die an den vier Ecken eines Quadrats angeordnet sind und welche über elektrische Leitungen mit dem Steuergerät verbunden oder verbindbar sind, wobei das Steuergerät eingerichtet ist,
ein elektrisches Wechselstromsignal zu erzeugen und an zwei der vier Kontaktelektroden abzugeben, so dass diese als Stromelektroden fungieren, und
ein elektrisches Wechselspannungssignal zwischen zwei der vier Kontaktelektroden aufzunehmen, so dass diese als Spannungselektroden fungieren,
wobei wenigstens zwei der Kontaktelektroden, die als Spannungselektroden fungieren, nicht gleichzeitig als Stromelektroden fungieren, wobei
das Steuergerät ferner dazu eingerichtet ist, ein elektrisches Wechselstromsignal nacheinander
- gleichzeitig an die erste und die zweite Kontaktelektrode,
- gleichzeitig an die erste und die dritte Kontaktelektrode,
- gleichzeitig an die erste und die vierte Kontaktelektrode,
- gleichzeitig an die zweite und die dritte Kontaktelektrode,
- gleichzeitig an die zweite und die vierte Elektrode und
- gleichzeitig an die dritte und vierte Kontaktelektrode abzugeben, wobei
das Steuergerät eine Recheneinheit umfasst, die eingerichtet ist, aus den mit verschiedenen Elektrodenkonfigurationen aufgenommenen Wechselspannungssignalen eine räumliche Impedanzverteilung des untersuchten Brustgewebes zu berechnen.

2. Bioimpedanz-Messgerät nach Anspruch 1, **dadurch gekennzeichnet, dass** der Messapplikator einen Trägerbereich mit armartigen Fortsätzen umfasst, an deren Enden die Kontaktelektroden angeordnet sind.

3. Bioimpedanz-Messgerät nach einem der Ansprüche 1 bis 2, **dadurch gekennzeichnet, dass** der Trägerbereich eine Öffnung umfasst, die an einem Mittelpunkt des regelmäßigen Polygons angeordnet ist, und eine reproduzierbare Positionierung des Messapplikators auf der Mamille einer zu vermessenden Brust zu ermöglichen.

4. Bioimpedanz-Messgerät nach Anspruch 2 oder 3, **dadurch gekennzeichnet, dass** der Trägerbereich aus einem flexiblen Material besteht.

5. Bioimpedanz-Messgerät nach einem der vorangehenden Ansprüche, **dadurch gekennzeichnet, dass** die Kontaktelektroden lösbar mit dem Messapplikator verbunden sind.

6. Bioimpedanz-Messgerät nach einem der vorangehenden Ansprüche, **dadurch gekennzeichnet, dass** das Steuergerät eingerichtet ist, das Wechselstromsignal mit veränderlicher Frequenz zu erzeugen.

7. Bioimpedanz-Messgerät nach einem der vorangehenden Ansprüche, **dadurch gekennzeichnet, dass** das Steuergerät eingerichtet ist, wechselweise unterschiedliche Kontaktelektroden als Stromelektroden anzusteuern.

8. Bioimpedanz-Messgerät nach einem der vorangehenden Ansprüche, **dadurch gekennzeichnet, dass** das Steuergerät eingerichtet ist, wechselweise unterschiedliche Kontaktelektroden als Spannungselektroden anzusteuern.

9. Bioimpedanz-Messgerät nach einem der vorangehenden Ansprüche, **dadurch gekennzeichnet, dass** die Recheneinheit eingerichtet ist, aus den bei verschiedenen Frequenzen aufgenommenen Wechselspannungssignalen eine räumliche Impedanzverteilung des untersuchten Brustgewebes zu berechnen.

10. Bioimpedanz-Messgerät nach einem der vorangehenden Ansprüche, **dadurch gekennzeichnet, dass** das Steuergerät eine Speichereinrichtung umfasst und eingerichtet ist, die berechnete Impedanzverteilung und/oder die bei verschiedenen Frequenzen und/oder mit verschiedenen Elektrodenkonfigurationen aufgenommenen Wechselspannungssignale zu speichern.

11. Bioimpedanz-Messgerät nach einem der vorangehenden Ansprüche, **dadurch gekennzeichnet, dass** das Steuergerät in einem fahrbaren Gehäuse angeordnet ist.

12. Bioimpedanz-Messgerät nach Anspruch 11, **dadurch gekennzeichnet, dass** das fahrbare Gehäuse einen Auslegerarm umfasst, entlang dessen die elektrischen Leitungen geführt oder führbar sind.

13. Bioimpedanz-Messgerät nach einem der vorangehenden Ansprüche, **dadurch gekennzeichnet, dass** das Bioimpedanz-Messgerät zwei Messapplikatoren umfasst.

14. Messapplikator eines Bioimpedanz-Messgeräts nach einem der Ansprüche 1 bis 5.

15. Verfahren zur Aufnahme eines Bioimpedanzprofils von menschlichen Brustgewebe, mit den Schritten:
- Applizieren eines Messapplikators mit vier Kontaktelektroden auf das zu untersuchende Brustgewebe,
- Einspeisen eines Wechselstromsignals über zwei der vier Kontaktelektroden, die als Stromelektroden fungieren, und
- Aufnehmen eines Wechselspannungssignals über zwei der vier Kontaktelektroden, die als Spannungselektroden fungieren,
wobei eine Frequenz des Wechselstromsignals über einen vorgegebenen Frequenzbereich variiert wird, und
wobei eine Funktion jeder der vier Kontaktelektroden zwischen Stromelektrode und Spannungselektrode gewechselt wird, wobei der Messapplikator vier an den Ecken eines Quadrats angeordnete Kontaktelektroden umfasst, und wobei
- in einem ersten Messintervall eine erste und eine zweite Kontaktelektrode als Stromelektrode fungieren, und eine dritte und eine vierte Kontaktelektrode als Spannungselektrode fungieren,
- in einem zweiten Messintervall die erste und die dritte Kontaktelektrode als Stromelektrode fungieren, und die zweite und die vierte Kontaktelektrode als Spannungselektrode fungieren,
- in einem dritten Messintervall die erste und die vierte Kontaktelektrode als Stromelektrode fungieren, und die zweite und die dritte Kontaktelektrode als Spannungselektrode fungieren,
- in einem vierten Messintervall die zweite und die dritte Kontaktelektrode als Stromelektrode fungieren, und die erste und die vierte Kontaktelektrode als Spannungselektrode fungieren,
- in einem fünften Messintervall die zweite und die vierte Kontaktelektrode als Stromelektrode fungieren, und die erste und die dritte Kontaktelektrode als Spannungselektrode fungieren, und
- in einem sechsten Messintervall die dritte und die vierte Kontaktelektrode als Stromelektrode fungieren, und die erste und die zweite Kontaktelektrode als Spannungselektrode fungieren, und wobei
mittels einer Recheneinheit aus den mit verschiedenen Elektrodenkonfigurationen aufgenommenen Wechselspannungssignalen eine räumliche Impedanzverteilung des untersuchten Brustgewebes berechnet wird.

16. Verfahren nach Anspruch 15, **dadurch gekennzeichnet, dass** die Frequenz des Wechselstromsignals in jedem Messintervall in mehreren Frequenzschritten in einem Frequenzbereich zwischen 1kHz und 1MHz variiert wird.

17. Verfahren nach Anspruch 16, **dadurch gekennzeichnet, dass** in jedem Messintervall und bei jedem Frequenzschritt eine Amplitude und eine Phase des Spannungssignals gemessen werden.

18. Verfahren nach einem der Ansprüche 15 bis 17, **dadurch gekennzeichnet, dass** die aufgenommenen Messwerte als Bioimpedanzprofil gespeichert werden.

19. Verfahren nach Anspruch 18, **dadurch gekennzeichnet, dass** das gespeicherte Bioimpedanzprofil mit einem zuvor aufgenommenen Bioimpedanzprofil des selben Gewebes verglichen wird.

20. Verfahren nach Anspruch 19, **dadurch gekennzeichnet, dass** Änderungen des Bioimpedanzprofils, welche ein vorgegebenes Maß übersteigen, ein Veränderungssignal erzeugt wird.

## Claims

1. Bioimpedance measuring device for measuring breast tissue, comprising a control unit and at least one measuring applicator having a first, a second, a third and a fourth contact electrode, which are arranged at the four corners of a square and which are or can be connected to the control unit via electrical lines, wherein the control unit is configured to
generating an alternating current electrical signal and outputting it to two of the four contact electrodes so that they function as current electrodes, and
receiving an alternating voltage electrical signal between two of the four contact electrodes so that they function as voltage electrodes,
wherein at least two of the contact electrodes functioning as voltage electrodes do not simultaneously function as current electrodes,
the control unit being further configured to apply an alternating electrical current signal in succession
- simultaneously to the first and second contact electrodes,
- simultaneously to the first and third contact electrodes,
- simultaneously to the first and fourth contact electrodes,
- simultaneously to the second and third contact electrodes,
- simultaneously to the second and fourth contact electrodes, and
- simultaneously to the third and fourth contact electrodes,
the control unit comprises a computing unit that is configured to calculate a spatial impedance distribution of the breast tissue under examination from the alternating voltage signals recorded with different electrode configurations.

2. Bioimpedance measuring device according to claim 1, **characterized in that** the measuring applicator comprises a carrier region with arm-like extensions, at the ends of which the contact electrodes are arranged.

3. Bioimpedance measuring device according to one of claims 1 to 2, **characterized in that** the carrier region comprises an opening which is arranged at a midpoint of the regular polygon, and to enable reproducible positioning of the measuring applicator on the nipple of a breast to be measured.

4. Bioimpedance measuring device according to claim 2 or 3, **characterized in that** the carrier region consists of a flexible material.

5. Bioimpedance measuring device according to one of the preceding claims, **characterized in that** the contact electrodes are detachably connected to the measuring applicator.

6. Bioimpedance measuring device according to one of the preceding claims, **characterized in that** the control unit is configured to generate the alternating current signal with variable frequency.

7. Bioimpedance measuring device according to one of the preceding claims, **characterized in that** the control unit is configured to alternately control different contact electrodes as current electrodes.

8. Bioimpedance measuring device according to one of the preceding claims, **characterized in that** the control unit is configured to alternately control different contact electrodes as voltage electrodes.

9. Bioimpedance measuring device according to one of the preceding claims, **characterized in that** the computing unit is configured to calculate a spatial impedance distribution of the examined breast tissue from the alternating voltage signals recorded at different frequencies.

10. Bioimpedance measuring device according to one of the preceding claims, **characterized in that** the control unit comprises a memory device and is configured to store the calculated impedance distribution and/or the alternating voltage signals recorded at different frequencies and/or with different electrode configurations.

11. Bioimpedance measuring device according to one of the preceding claims, **characterized in that** the control unit is arranged in a mobile housing.

12. Bioimpedance measuring device according to claim 11, **characterized in that** the mobile housing comprises an extension arm along which the electric lines are guided or can be guided.

13. Bioimpedance measuring device according to one of the preceding claims, **characterized in that** the bioimpedance measuring device comprises two measuring applicators.

14. Measuring applicator of a bioimpedance measuring device according to one of claims 1 to 5.

15. Method for recording a bioimpedance profile of human breast tissue, comprising the steps of:
applying a measuring applicator having four contact electrodes to the breast tissue to be examined,
feeding an alternating current signal via two of the four contact electrodes, which act as current electrodes, and
recording an alternating voltage signal via two of the four contact electrodes, which act as voltage electrodes,
wherein a frequency of the alternating current signal is varied over a predetermined frequency range, and
wherein a function of each of the four contact electrodes is alternated between current electrode and voltage electrode, wherein the measurement applicator comprises four contact electrodes arranged at the corners of a square, and wherein
- in a first measuring interval, a first and a second contact electrode function as current electrode and a third and a fourth contact electrode function as voltage electrode,
- in a second measuring interval, the first and the third contact electrode function as current electrode and the second and the fourth contact electrode function as voltage electrode,
- in a third measuring interval, the first and fourth contact electrodes act as current electrodes and the second and third contact electrodes act as voltage electrodes,
- in a fourth measuring interval, the second and third contact electrodes act as current electrodes and the first and fourth contact electrodes act as voltage electrodes,
- in a fifth measuring interval, the second and fourth contact electrodes function as current electrodes, and the first and third contact electrodes function as voltage electrodes, and
- in a sixth measuring interval, the third and fourth contact electrodes function as current electrodes, and the first and second contact electrodes function as voltage electrodes, and wherein
a spatial impedance distribution of the examined breast tissue is calculated by means of a computing unit from the alternating voltage signals recorded with different electrode configurations.

16. Method according to claim 15, **characterized in that** the frequency of the alternating current signal is varied in each measuring interval in a plurality of frequency steps in a frequency range between 1kHz and 1MHz.

17. Method according to claim 16, **characterized in that** an amplitude and a phase of the voltage signal are measured in each measuring interval and at each frequency step.

18. Method according to one of claims 15 to 17, **characterized in that** the measured values recorded are stored as a bioimpedance profile.

19. Method according to claim 18, **characterized in that** the stored bioimpedance profile is compared with a previously recorded bioimpedance profile of the same tissue.

20. Method according to claim 19, **characterized in that**, when changes in the bioimpedance profile which exceed a predetermined level, a change signal is generated.

## Revendications

1. Dispositif de mesure de la bioimpédance pour mesurer le tissu mammaire, comprenant une unité de contrôle et au moins un applicateur de mesure comprenant une première, une deuxième, une troisième et une quatrième électrode de contact, qui sont disposées aux quatre coins d'un carré et qui sont ou peuvent être connectées à l'unité de contrôle par des lignes électriques, dans lequel l'unité de contrôle est configurée pour
générer un signal électrique de courant alternatif et l'envoyer à deux des quatre électrodes de contact afin qu'elles fonctionnent comme des électrodes de courant, et
recevoir un signal électrique de tension alternative entre deux des quatre électrodes de contact de manière à ce qu'elles fonctionnent comme des électrodes de tension,
dans lequel au moins deux des électrodes de contact fonctionnant comme des électrodes de tension ne fonctionnent pas simultanément comme des électrodes de courant,
l'unité de contrôle est en outre configurée pour appliquer un signal de courant électrique alternatif en succession
- simultanément aux première et deuxième électrodes de contact,
- simultanément aux première et troisième électrodes de contact,
- simultanément aux première et quatrième électrodes de contact,
- simultanément aux deuxième et troisième électrodes de contact,
- simultanément aux deuxième et quatrième électrodes de contact, et
- simultanément aux troisième et quatrième électrodes de contact,
l'unité de contrôle comprend une unité de calcul configurée pour calculer une distribution d'impédance spatiale du tissu mammaire examiné à partir des signaux de tension alternative enregistrés avec différentes configurations d'électrodes.

2. Dispositif de mesure de la bioimpédance selon la revendication 1, **caractérisé en ce que** l'applicateur de mesure comprend une région porteuse avec des extensions en forme de bras, aux extrémités desquelles sont disposées les électrodes de contact.

3. Dispositif de mesure de la bioimpédance selon l'une des revendications 1 à 2, **caractérisé en ce que** la région porteuse comprend une ouverture qui est disposée à un point médian du polygone régulier, et pour permettre un positionnement reproductible de l'applicateur de mesure sur le mamelon d'un sein à mesurer.

4. Dispositif de mesure de la bioimpédance selon la revendication 2 ou 3, **caractérisé en ce que** la région porteuse est constituée d'un matériau flexible.

5. Dispositif de mesure de la bioimpédance selon l'une des revendications précédentes, **caractérisé en ce que** les électrodes de contact sont reliées de manière amovible à l'applicateur de mesure.

6. Dispositif de mesure de la bioimpédance selon l'une des revendications précédentes, **caractérisé en ce que** l'unité de commande est configurée pour générer le signal de courant alternatif avec une fréquence variable.

7. Dispositif de mesure de la bioimpédance selon l'une des revendications précédentes, **caractérisé en ce que** l'unité de commande est configurée pour commander alternativement différentes électrodes de contact en tant qu'électrodes de courant.

8. Dispositif de mesure de la bioimpédance selon l'une des revendications précédentes, **caractérisé en ce que** l'unité de commande est configurée pour commander alternativement différentes électrodes de contact en tant qu'électrodes de tension.

9. Dispositif de mesure de la bioimpédance selon l'une des revendications précédentes, **caractérisé en ce que** l'unité de calcul est configurée pour calculer une distribution d'impédance spatiale du tissu mammaire examiné à partir des signaux de tension alternatifs enregistrés à différentes fréquences.

10. Dispositif de mesure de la bioimpédance selon l'une des revendications précédentes, **caractérisé en ce que** l'unité de commande comprend un dispositif de mémoire et est configurée pour stocker la distribution d'impédance calculée et/ou les signaux de tension alternative enregistrés à différentes fréquences et/ou avec différentes configurations d'électrodes.

11. Dispositif de mesure de la bioimpédance selon l'une des revendications précédentes, **caractérisé en ce que** l'unité de commande est disposée dans un boîtier mobile.

12. Dispositif de mesure de la bioimpédance selon la revendication 11, **caractérisé en ce que** le boîtier mobile comprend un bras d'extension le long duquel les lignes électriques sont guidées ou peuvent être guidées.

13. Dispositif de mesure de la bioimpédance selon l'une des revendications précédentes, **caractérisé en ce que** le dispositif de mesure de la bioimpédance comprend deux applicateurs de mesure.

14. Applicateur de mesure d'un dispositif de mesure de la bioimpédance selon l'une des revendications 1 à 5.

15. Méthode d'enregistrement d'un profil de bioimpédance d'un tissu mammaire humain, comprenant les étapes suivantes :
appliquer un applicateur de mesure comportant quatre électrodes de contact sur le tissu mammaire à examiner,
envoyer un signal de courant alternatif via deux des quatre électrodes de contact, qui agissent comme des électrodes de courant, et
enregistrer un signal de tension alternatif par l'intermédiaire de deux des quatre électrodes de contact, qui agissent comme des électrodes de tension,
dans lequel la fréquence du signal de courant alternatif varie sur une plage de fréquences prédéterminée, et
dans lequel la fonction de chacune des quatre électrodes de contact est alternée entre l'électrode de courant et l'électrode de tension, dans lequel l'applicateur de mesure comprend quatre électrodes de contact disposées aux coins d'un carré, et dans lequel
- dans un premier intervalle de mesure, une première et une deuxième électrode de contact fonctionnent comme électrode de courant et une troisième et une quatrième électrode de contact fonctionnent comme électrode de tension,
- dans un deuxième intervalle de mesure, la première et la troisième électrode de contact fonctionnent comme électrode de courant et la deuxième et la quatrième électrode de contact fonctionnent comme électrode de tension,
- dans un troisième intervalle de mesure, les première et quatrième électrodes de contact agissent comme des électrodes de courant et les deuxième et troisième électrodes de contact agissent comme des électrodes de tension,
- dans un quatrième intervalle de mesure, les deuxième et troisième électrodes de contact agissent en tant qu'électrodes de courant et les première et quatrième électrodes de contact agissent en tant qu'électrodes de tension,
- dans un cinquième intervalle de mesure, les deuxième et quatrième électrodes de contact fonctionnent comme des électrodes de courant et les première et troisième électrodes de contact fonctionnent comme des électrodes de tension, et
- dans un sixième intervalle de mesure, les troisième et quatrième électrodes de contact fonctionnent comme des électrodes de courant et les première et deuxième électrodes de contact fonctionnent comme des électrodes de tension, et dans lequel
une distribution d'impédance spatiale du tissu mammaire examiné est calculée au moyen d'une unité de calcul à partir des signaux de tension alternative enregistrés avec différentes configurations d'électrodes.

16. Méthode selon la revendication 15, **caractérisé en ce que** la fréquence du signal de courant alternatif varie dans chaque intervalle de mesure en plusieurs étapes de fréquence dans une gamme de fréquences comprise entre 1kHz et 1MHz.

17. Procédé selon la revendication 16, **caractérisé en ce qu'**une amplitude et une phase du signal de tension sont mesurées dans chaque intervalle de mesure et à chaque pas de fréquence.

18. Procédé selon l'une des revendications 15 à 17, **caractérisé en ce que** les valeurs mesurées enregistrées sont stockées sous la forme d'un profil de bioimpédance.

19. Procédé selon la revendication 18, **caractérisé en ce que** le profil de bioimpédance enregistré est comparé à un profil de bioimpédance précédemment enregistré du même tissu.

20. Procédé selon la revendication 19, **caractérisé en ce que**, lorsque les changements dans le profil de bioimpédance dépassent un niveau prédéterminé, un signal de changement est généré.
